# EUROPEAN PATENT APPLICATION

(11) **EP 4 282 415 A1**
(43) Date of publication of application: **29.11.2023**
(21) Application number: 22386032.1
(22) Date of filing: 26.05.2022
(51) Int. Cl.: A61K 31/4439

(54) **A STABLE TABLET COMPOSITION OF AXITINIB**

(71) Applicant: Genepharm S.A., 15351 Pallini (GR)
(72) Inventor: Xenogiorgis, Vasileios, 17676 Kallithea Athens (GR); Mpenekis, Vasilis, 16341 Ilioupoli Athens (GR)
(74) Representative: Roukounas, Dimitrios

(57) **Abstract**

A stable tablet composition of Axitinib comprising crystalline form IV Axitinib in the range of 1 to 10%; reducing sugar(s) in the range of 0.5 to 15%; at least one pharmaceutical excipient(s) selected from disintegrant, diluent, binder, lubricant, antioxidant and glidant; and wherein the % is weight by weight of the tablet composition and wherein the composition is bioequivalent to axitinib product (Inlyta^{®}).

## Description

### FIELD OF THE INVENTION

The present invention relates to a tablet composition of Axitinib which is chemically and physically stable for at least three months.

### BACKGROUND OF THE INVENTION

Axitinib or N-methyl-2-( {3-[(E)-2-pyridin-2-ylethenyl]-IH-indazol-6-yl} sulfanyl) benzamide is an inhibitor of several tyrosine kinases involved in angiogenesis, particularly of VEGFR tyrosine kinases.

Axitinib is marketed as Inlyta^{®} by Pfizer under the brand name Inlyta^{®} and claimed in European patent number EP1218348B1 (by M/S Agouron Pharmaceuticals), referred to herein as EP '348). Inlyta^{®} is marketed as immediate release film-coated tablets, in different strengths (1, 3, 5 and 7 mg). The product is approved for the treatment of advanced kidney cancer (advanced Renal cell carcinoma) in adults, when another medicine such as Sunitinib or a cytokine is no longer stopping disease from progressing.

Several crystalline forms of axitinib characterized by characteristic X-ray diffraction peaks have been disclosed in PCT Publication Numbers WO2006048751 (by M/S Pfizer Inc., referred to herein as WO '751) and WO2008122858 (by M/S Pfizer Products Inc., referred to herein as WO '858).

Axitinib is a BCS class II compound exhibiting low solubility. It is highly soluble only in the gastric at pH 1. 7. Inlyta^{®} contains the crystalline axitinib form XLI. Form XLI is the most thermodynamically stable polymorphic form. Crystalline Form IV is known to be less photo stable compared to form XLI but is 2-3 times more soluble in water.

Crystalline Form IV has characteristic X-ray diffraction peaks at about 8.9,12.0,14.6,15.2, 15.7,17.8, 19.2, 20.5, 21.6, 23.2, 24.2, 24.8, 26.2 and 27.5 ± 0.1 degrees 2θ, when measured with Cu KαI radiation. Crystalline Form XLI has characteristic X-ray diffraction peaks at about 6.0, 11.5, 11.9, 12.5, 12.9, 14.9, 15.6, 16.2, 16.5, 17.9, 19.9, 20.7, 21.0, 21.6, 22.4, 22.8, 23.1, 24.2, 24.5, 25.0, 25.3, 25.6, 25.9, 26.4, 26.9, 27.7, 28.0, 28.1, 28.5, 29.9, 30.9, 31.5, 32.9, 33.2, 34.8, 35.0, 36.1 ± 0.1 degrees 2θ.

Compositions of Axitinib are disclosed in PCT Publication Number WO2013046133 wherein the core is coated with a coating comprising metal oxide.

Formulation development with axitinib Form IV presented difficulties during bioequivalence study with marketed product Inlyta^{®}. A prototype comprising axitinib form IV showed a dissolution profile similar to Inlyta^{®} using FDA dissolution method, but higher bioavailability than Inlyta^{®} in vivo.

PCT Publication Number WO2020/225413 (by M/S Synthon, referred to herein as WO'413) teaches pharmaceutical compositions of Axitinib comprising crystalline form IV but there is no disclosure of the bioequivalence of the product with regards to Inlyta^{®}.

It is desirable to have a tablet composition comprising axitinib form IV that is stable and is bioequivalent to Inlyta^{®}.

### OBJECT OF THE INVENTION

The object of the present invention is to provide stable tablet composition of Axitinib.

### SUMMARY OF THE INVENTION

A stable tablet composition of Axitinib comprising
(a) crystalline form IV Axitinib in the range of 1 to 10%;
(b) reducing sugar(s) in the range of 0.5 to 15%;
(c) at least one pharmaceutical excipient(s) selected from disintegrant, diluent, lubricant, antioxidant and glidant; and
wherein the % is weight by weight of the tablet composition and wherein the composition is bioequivalent to axitinib product (Inlyta^{®}).

### BRIEF DESCRIPTION OF THE DRAWINGS

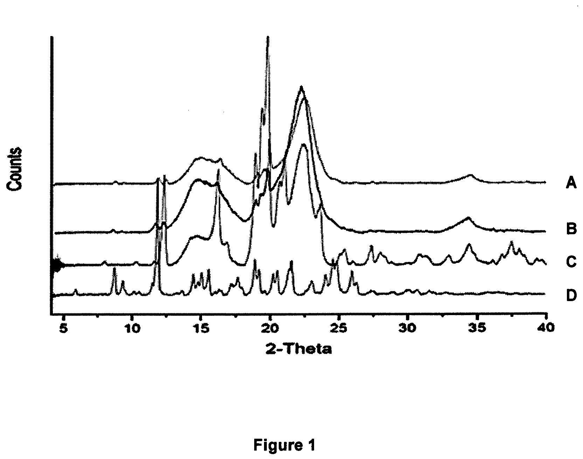

Figure 1 shows the X ray diffraction pattern of axitinib form IV in the stable tablet composition of the present invention. Crystalline form IV is retained in the tablets for three months at 40°C/75%RH.

### DESCRIPTION OF THE INVENTION

The present invention provides pharmaceutical compositions comprising crystalline form IV of Axitinib which are stable and bioequivalent to axitinib product (Inlyta^{®}).

An Axitinib tablet composition comprising axitinib form IV is found to be bioequivalent to Inlyta^{®} when it exhibits a dissolution rate between 30% and 70% in 30 minutes when tested in 900 ml 0.01 N hydrochloric acid pH 2.0 at 37°C, 75 rpm in a USP apparatus II.

Different polymorphic forms of compounds influence stability, dissolution and bioequivalence of a drug product. Like in case of Axitinib, bioequivalence studies failed when form IV was used to prepare tablet compositions. This could be probably due to the higher solubility of axitinib form IV as compared to form XLI.

The present invention is based on the surprising finding that a composition of Axitinib comprising crystalline form IV can be stabilized and made bioequivalent by selecting and optimising one excipient in a composition.

The present inventors found that when percent of reducing sugar was optimized in a composition comprising Axitinib Form IV, the composition is bioequivalent to Inlyta^{®}.

Although the load of axitinib form IV in the tablet composition of the present invention is small, the use of lower percentage of reducing sugar had an impact on the bioavailability of the tablet.

According to one embodiment of the present invention is a stable tablet composition comprising 1 to 10% of crystalline form IV Axitinib; 0.5 to 15% reducing sugar(s) and at least one pharmaceutically acceptable excipient(s) selected form disintegrant, diluent, binder, lubricant, antioxidant and glidant; and wherein the % is weight by weight of the tablet composition and wherein the composition is bioequivalent to axitinib product (Inlyta^{®}).

Axitinib form IV is characterized by X-ray diffraction at about 8.9, 12.0, 14.6, 15.2, 15.7, 17.8, 19.1, 20.6, 21.6, 23.2, 24.2, 24.9, 26.1 and 27.5 ± 0.1 degrees 2θ, when measured with Cu Kal radiation.

The stable tablet composition of the present invention retains Axitinib crystalline form IV after storage at 25°C/60%RH for at least three months.

The stable tablet composition of the present invention retains Axitinib crystalline form IV after storage at 40°C/75%RH for at least three months.

The particle size of Axitinib used in the present invention is d90 less than or equal to 50 microns.

The reducing sugar to be used in the present invention may be selected from lactose, maltose, fructose, galactose, ribose, arabinose, glucose and xylose.

The inventors have found that the disintegration time of the stable tablet composition of the present invention is inversely proportional to the percentage of reducing sugar when measured by a disintegration apparatus according to EP 2.9.1 disintegration of tablets and capsules.

The inventors have found that the dissolution time of the stable tablet composition of the present invention is directly proportional to the percentage of reducing sugar when measured by dissolution USP apparatus II according to EP 2.9.3 dissolution test for solid dosage forms.

The total weight of the core tablet of the invention is typically between 50 and 900 mg and preferably between 90 and 800 mg.

The dissolution method used in the current invention comprises dissolving axitinib drug product at 37°C in 900 ml of hydrochloric acid 0.01 N having pH 2.0 and stirring it using a USP apparatus having paddles II and 75 rpm.

The stable tablet composition of the invention may comprise pharmaceutical excipient(s) selected from disintegrants, diluents, binders, lubricants, antioxidants and glidants.

The disintegrants to be used in the present invention may be selected from croscarmellose sodium, crospovidone, low-substituted hydroxypropylcellulose, sodium starch glycolate or a mixture thereof. The disintegrants may be in the range of 0.05 to 1% weight by weight of the tablet composition.

The inventors have found that the dissolution time of the stable tablet composition of the present invention is directly proportional to the percentage of disintegrant when measured by dissolution USP apparatus II according to EP 2.9.3 dissolution test for solid dosage forms.

The diluents to be used in the present invention may be selected from sucrose, calcium carbonate, mannitol, cellulose, maltose, sorbitol, starch or a mixture thereof. The diluents may be in the range of 73 to 99% weight by weight of the tablet composition.

The binders to be used in the present invention may be selected from microcrystalline cellulose (MCC), sodium carboxymethylcellulose, polyvinyl pyrrolidone (PVP), copovidone, polyvinyl pyrrolidone- vinyl acetate (PVP/VA) copolymer, hydroxypropylcellulose, hydroxypropyl methylcellulose, ethyl cellulose, hypromellose or a mixture thereof.

The lubricants to be used in the present invention may be selected from stearic acid. Calcium stearate, sodium stearyl fumarate or a mixture thereof. The lubricants may be in the range of 0.2 to 1% weight by weight of the tablet composition.

The antioxidants to be used in the present invention may be selected from ascorbyl palmitate, butyl hydroxyanisole (BHA), butyl hydroxytoluene (BHT), tocopherols and the like.

The glidants to be used in the present invention may be selected from colloidal silicon dioxide, talc or a mixture thereof.

The hardness of the stable tablet of the present invention is found to be inversely proportional to the core tablet weight wherein the Hardness test is measured by hardness tester according to EP 2.9.8 resistance to crushing of tablets.

The tablets may be optionally coated by a film-coat. The coating may be selected from hypromellose based coating materials.

Coating is done using any conventional coating technique known in the art, such as spray coating in a conventional coating pan or fluidized bed processor or dip coating.

The tablet composition according to the present invention is packaged in primary packaging material, e.g., blisters. The tablet composition of the present invention is preferably packaged in PVC/PE/PVDC blisters (triplex) or Alu-Alu blisters.

According to another embodiment of the present invention is the process for the preparation of stable tablet composition comprising crystalline form IV of Axitinib may be selected form wet granulation, dry granulation, direct compression, slugging,

The preferred process for the preparation of stable tablet composition comprising crystalline form IV of Axitinib is direct compression.

The following examples illustrate preferred embodiments in accordance with the present invention without limiting the scope of the invention.

### EXAMPLES

### Comparative Example: Not within the scope of the Present Invention

| **AXT0220-07-7** | **7 mg** | |
|---|---|---|
| **Composition** | **mg/tab** | **%** |
| **Axitinib (non-micronized)** | 7.00 | 7.00 |
| **MCC 102** | 57.25 | 57.25 |
| **Lactose Monohydrate** | 32.00 | 32.00 |
| **Croscarmellose sodium** | 3.00 | 3.00 |
| Intragranule Weight | 99.25 | 99.25 |
| **Magnesium Stearate** | 0.75 | 0.75 |
| Core Tablet Weight | **100.00** | **100.00** |

### Example 1 Axitinib tablets of the present invention

| | **mg/tab** | **%** |
|---|---|---|
| Axitinib Form IV d90 = 30µm | 7.00 | 4.67 |
| MCC 102 | 134.13 | 89.42 |
| Lactose monohydrate | 7.50 | 5.00 |
| Croscarmellose sodium | 0.37 | 0.25 |
| Magnesium stearate | 1.00 | 0.67 |
| - | - | - |
| - | - | - |
| **Total core** | **150.00** | **100.00** |
| Opadry II Red (HPMC based) | | 3.00 |
| | **154.50** | |

### Physical attributes of tablets:

| | |
|---|---|
| Average Weight (mg) | 150.15 |
| DT (min: s) | 10'06" |
| Hardness (N) | 206.00 |

Axitinib Crystalline form IV is retained in the tablets for three months at 40°C/75%RH, when measured by XRD. Fig.1 shows the XRD pattern of Axitinib 7mg FC tabs Lot: AXT0220-26-7 3M 40/75, packed in HDPE bottle with dessicant (A), Axitinib 7mg FC tabs Lot: AXT0220-26-7 zero time (B), Axitinib Processed Placebo of batch 7 (C) and Axitinib API Form IV Lot: XA0010418 (D)

### Example 2 Axitinib tablets of the present invention

| | **mg/tab** | **%** |
|---|---|---|
| Axitinib Form IV d90=30µm | 7.00 | 4.67 |
| MCC 101 | 65.00 | 43.33 |
| Lactose monohydrate | 47.00 | 31.33 |
| Purified water | qs | |
| MCC 102 | 28.00 | 18.67 |
| Croscarmellose sodium | 2.00 | 1.33 |
| Magnesium stearate | 1.00 | 0.67 |
| **Total core** | **150.00** | **100.00** |
| Opadry II Red (HPMC based) | | 3.00 |
| | **154.50** | |

### Example 3 Chemical stability of composition of Example 1 with assay values

| | | |
|---|---|---|
| **Related substances (%)** | | |
| Zero time | 4 months 25°/60RH% | 4 months 40°/75RH% |
| Total impurities: 0.34 | Total impurities: 0.32 | Total impurities: 0.33 |
| Max known impurity: 0.07 | Max known impurity: 0.07 | Max known impurity: 0.07 |
| Max unknown impurity: 0.07 | Max unknown impurity: 0.07 | Max unknown impurity: 0.07 |

| **Assay (%)** | | |
|---|---|---|
| Zero time | 4 months 25°/60RH% | 4 months 40°/75RH% |
| 99.7 | 100.0 | 99.6 |

### Example 4: Dissolution Profiles

| | **Example 1** | **Example 1** | **Example 2** | **Example 2** |
|---|---|---|---|---|
| METHOD | HCl 0.1N 50 rpm | HCl 0.01N 75 rpm | HCl 0.1N 50 rpm | HCl 0.01N 75 rpm |
| 10 | 49.0 | 7.1 | 93.7 | 28.9 |
| 15 | 63.0 | 26.1 | 95.7 | 55.3 |
| 30 | 82.2 | 43.6 | 97.1 | 71.6 |
| 45 | | 55.0 | | 79.6 |
| 60 | | 62.9 | | 84.6 |
| **Assay (%)** | **99.7** | | **97.1** | |

### Example 5-13:

| | **Example 5** | | **Example 6** | | **Example 7** | | **Example 8** | |
|---|---|---|---|---|---|---|---|---|
| Composition | mg/tab | % | mg/tab | % | mg/tab | % | mg/tab | % |
| Axitinib | 7.00 | 4.67 | 7.00 | 4.67 | 7.00 | 4.67 | 7.00 | 4.67 |
| Lactose Monohydrate | 13.50 | 9.00 | 1.50 | 1.00 | 13.50 | 9.00 | 1.50 | 1.00 |
| MCC 102 | 128.35 | 85.57 | 140.35 | 93.57 | 127.90 | 85.27 | 139.90 | 93.27 |
| Croscarmellose Sodium | 0.15 | 0.10 | 0.15 | 0.10 | 0.60 | 0.40 | 0.60 | 0.40 |
| Magnesium Stearate | 1.00 | 0.67 | 1.00 | 0.67 | 1.00 | 0.67 | 1.00 | 0.67 |
| Tablet Weight | 150.00 | 100.00 | 150.00 | 100.00 | 150.00 | 100.00 | 150.00 | 100.00 |

| | **Example 9** | | **Example 10** | | **Example 11** | |
|---|---|---|---|---|---|---|
| Composition | mg/tab | % | mg/tab | % | mg/tab | % |
| Axitinib | 7.00 | 3.89 | 7.00 | 3.89 | 7.00 | 3.89 |
| Lactose Monohydrate | 9.00 | 5.00 | 16.20 | 9.00 | 1.80 | 1.00 |
| MCC 102 | 162.55 | 90.31 | 155.62 | 86.46 | 170.02 | 94.46 |
| Croscarmellose Sodium | 0.45 | 0.25 | 0.18 | 0.10 | 0.18 | 0.10 |
| Magnesium Stearate | 1.00 | 0.56 | 1.00 | 0.56 | 1.00 | 0.56 |
| Tablet Weight | 180.00 | 100.00 | 180.00 | 100.00 | 180.00 | 100.00 |

| | **Example 12** | | **Example 13** | |
|---|---|---|---|---|
| Composition | mq/tab | % | mg/tab | % |
| Axitinib | 7.00 | 3.89 | 7.00 | 3.89 |
| Lactose Monohydrate | 16.20 | 9.00 | 1.80 | 1.00 |
| MCC 102 | 155.08 | 86.16 | 169.48 | 94.16 |
| Croscarmellose Sodium | 0.72 | 0.40 | 0.72 | 0.40 |
| Magnesium Stearate | 1.00 | 0.56 | 1.00 | 0.56 |
| Tablet Weight | 180.00 | 100.00 | 180.00 | 100.00 |

## Claims

1. A stable tablet composition of Axitinib comprising
(a) crystalline form IV Axitinib in the range of 1 to 10%;
(b) reducing sugar(s) in the range of 0.5 to 15%;
(c) at least one pharmaceutical excipient(s) selected from disintegrants, diluents, binders, lubricants, antioxidants and glidants; and
wherein the % is weight by weight of the tablet composition and wherein the composition is bioequivalent to axitinib product (Inlyta^{®}).

2. A stable tablet composition as claimed in claim 1 wherein the tablet retains crystalline form IV for at least three months at 25°C/60%RH with regards to crystallinity.

3. A stable tablet composition as claimed in claim 1 wherein the tablet retains crystalline form IV for at least three months at 40°C/75%RH with regards to crystallinity.

4. A stable tablet composition as claimed in claim 1 wherein the Axitinib is with d90 less than or equal to 50 microns.

5. A stable tablet composition as claimed in claim 1 wherein the reducing sugar is selected from lactose, maltose, fructose, galactose, ribose, arabinose, glucose and xylose.

6. A stable tablet composition as claimed in claim 1 comprising disintegrant is in the range of 0.05 to 1% weight by weight of the tablet composition.

7. A stable tablet composition as claimed in claim 1 comprising diluent is in the range of 73 to 99% weight by weight of the tablet composition.

8. A stable tablet composition as claimed in claim 1 comprising lubricant is in the range of 0.2 to 1% weight by weight of the tablet composition.

9. A stable tablet composition as claimed in claim 1 further comprising film coating selected from hypromellose based coating materials.

10. A stable tablet composition as claimed in claim 1 wherein the tablet hardness is inversely proportional to the core tablet weight when measured by hardness tester according to EP 2.9.8 resistance to crushing of tablets.

11. A stable tablet composition as claimed in claim 1 wherein the disintegration time is inversely proportional to the percentage of reducing sugar when measured by a disintegration apparatus according to EP 2.9.1 disintegration of tablets and capsules.

12. A stable tablet composition as claimed in claim 1 wherein the dissolution time is directly proportional to the percentage of reducing sugar when measured by dissolution USP apparatus II according to EP 2.9.3 dissolution test for solid dosage forms.

13. A stable tablet composition as claimed in claim 1 wherein the dissolution time is directly proportional to the percentage of disintegrant when measured by dissolution USP apparatus II according to EP 2.9.3 dissolution test for solid dosage forms.
